# EUROPEAN PATENT SPECIFICATION

(11) **EP 1 261 361 B1**
(45) Date of publication and mention of the grant of the patent: **14.06.2006**
(21) Application number: 01910913.1
(22) Date of filing: 16.02.2001
(51) Int. Cl.: A61K 38/00, A61P 37/00, A61P 25/00, A61K 38/16

(54) **ORAL, NASAL AND PULMONARY DOSAGE FORMULATIONS OF COPOLYMER 1 (GLATIRAMER ACETATE)**
FORMULIERUNGEN VON COPOLYMER 1 (GLATIRAMERACETAT) ZUR ORALEN, NASALEN UND PULMONALEN VERABREICHUNG
FORMES POSOLOGIQUES A ADMINISTRATION PAR VOIE ORALE, NASALE ET PULMONAIRE DU COPOLYMERE 1 (ACETATE DE GLATIRAMER)

(30) Priority: 18.02.2000 US 183666 P; 18.02.2000 US 507188
(43) Date of publication of application: 04.12.2002
(73) Proprietor: YEDA RESEARCH AND DEVELOPMENT COMPANY, LTD., Rehovot 7 (IL)
(72) Inventor: GILBERT, Adrian, Kfar Sava 44448 (IL); RIVEN-KREITMAN, Rivka, 44288 Kfar-Sava (IL); LINENBERG, Milka, 40600 Tel-Mond (IL); COHEN-VERED, Sharon, Kfar Sava 44502 (IL); JOUBRAN, Remon, F., Ramle 72211 (IL)
(74) Representative: Vossius & Partner
(86) International application number: PCT/US2001/005198
(87) International publication number: WO 2001/060392

(56) References cited:
- WO-A-98/30227
- WO-A1-00/05250
- WO-A1-00/20010
- US-A- 5 800 808
- HARRISON L C ET AL: "Antigen-specific therapy for autoimmune disease" CURRENT OPINION IN IMMUNOLOGY, CURRENT BIOLOGY LTD, XX, vol. 12, no. 6, 1 December 2000 (2000-12-01), pages 704-711, XP004257746 ISSN: 0952-7915
- DATABASE HCAPLUS [Online] TARCIC ET AL.: 'Copolymer I (copaxone) from an idea to a drug for treatment of multiple sclerosis', XP002939996 Retrieved from STN Database accession no. 1997:333270 & KIM., HANDASA KIM. vol. 281, no. 14, 1997, pages 16 - 18
- REILLY W.J., JR.: 'Pharmaceutical necessities' REMINGTON: THE SCIENCE AND PRACTICE OF PHARMACY vol. 2, 1995, pages 1380 - 1416, XP002939997
- O'CONNOR ROBERT ET AL.: 'Powders' REMINGTON: THE SCIENCE AND PRACTICE OF PHARMACY vol. 2, 1995, pages 1598 - 1614, XP002939998
- PORTER STRUART: 'Coating of pharmaceutical dosage forms' REMINGTON: THE SCIENCE AND PRACTICE OF PHARMACY vol. 2, 1995, pages 1650 - 1659, XP002939999
- DATABASE MEDLINE [Online] INSTITUTO DI CLINICA NEUROLOGICA, UNIVERSIT'A, TRIESTE, ITALY CAZZATO ET AL.: 'Treatment of multiple sclerosis. The present and the future. Study group on diagnosis and therapy of multiple sclerosis', XP002940000 Retrieved from STN Database accession no. 2000060325 & RECENT PROGRESSI IN MEDICINA vol. 90, no. 10, October 1999, pages 538 - 544
- DATABASE HCAPLUS [Online] DEPARTMENT OF CLINICAL IMMUNOLOGY, AARHUS UNIVERSITY HOSPITAL AARHUS, DENMARK FRIDKIS-HARELI ET AL.: 'Synthetic peptides that inhibit binding of the collagen type II 261-273 epitope to rheumatoid arthritis-associated HLA-DR1 and - DR4 molecules and collagen-specific T-cell responses', XP002940393 Retrieved from STN Database accession no. 2000:455053 & HUMAN IMMUNOLOGY vol. 61, no. 7, 2000, pages 640 - 650
- DATABASE HCAPLUS [Online] DEPARTMENT OF PHARMACEUTICAL TECHNOLOGY, GULHANE MILITARY MEDICAL ACADEMY, ANKARA, 06018, TURKEY KEPSUTLU ET AL.: 'Evaluation of chitosan used as an excipient in tablet formulations', XP002940394 Retrieved from STN Database accession no. 1999:590411 & ACTA POL. PHARM. vol. 56, no. 3, 1999, pages 227 - 235

## Description

The present invention provides oral, nasal and pulmonary dosage formulations of Copolymer 1. COPAXONEO is the brand name for glatiramer acetate (also known as Copolymer 1). Glatiramer acetate (GA), the active ingredient of COPAXONE®, consists of the acetate salts of synthetic polypeptides, containing four naturally occurring amino acids: L-glutamic acid, L-alanine, L-tyrosine, and L-lysine with average molar fractions of [L-Glu: 0:129-0.153; L-Ala: 0.392-0.462; L-Tyr: 0.086-0.100; L-Lys: 0.300-0.374] respectively. The average molecular weight of glatiramer acetate is 4,700 - 11,000 daltons. Chemically, glatiramer acetate is designated L-glutamic acid polymer with L-alanine, L-lysine and L-tyrosine, acetate (salt). Its structural formula is:

(Glu, Ala, Lys, Tyr)·_{X}CH₂COOH

(C₅H₃NO₄C₃H₂NO₂.C₂H₁₄N₄₂O₂.C₉H₁₄NO₂)₃.XC₂H₄O₂

CAS - 147245-92-9

("Copaxone", Physician's Desk Reference, (2000), Medical Economics Co., Inc., (Montvale, NJ), 3115.) Glatiramer acetate is also written as: poly [L-Glu¹³⁻¹⁵, L-Ala³⁹⁻⁴⁶, L-Tyr^{8.6-10}, L-Lys³⁰³⁷].n CH₃COOH. Copolymer 1 is widely believed to be effective in treating a variety of immune system_conditions. A common method of administering the drug is subcutaneous injection. However, such administration often results in injection site reactions such as irritation, hypersensitivity, inflammation and pain. In addition, it tends to be difficult to persuade patients to adhere to the prescribed dosing regimes. To overcome these difficulties, oral, nasal and pulmonary dosage formulations are desirable. WO 98/30227 relates to pharmaceutical compositions comprising Copolymer 1 useful for the treatment of multiple sclerosis by ingestion or inhalation. This invention provides compositions of and processes for creating solid, semisolid and aqueous dosage forms intended for oral, nasal and pulmonary administration.

Autoimmune diseases occur when a mammal's immune system fails to recognize some of the mammal's own tissues as "self" and attacks them as "foreign". Normally, self-tolerance is developed early by developmental events within the immune system that prevent the mammal's T cell and B cells from reacting with the mammal's own tissues. Major Histocompatibility Complex (MHC) cell surface proteins help regulate these early immune responses by binding to and presenting processed peptides to T cells.

When this self-tolerance breaks down, autoimmune diseases develop. Now, the mammal's own tissues and proteins are recognized as "antigens" and are attacked by the mammal's immune system. For example, multiple sclerosis is believed to be an autoimmune disease that occurs when the immune system attacks the myelin sheath. This sheath is thought to insulate and protect the nerves. - The disease is a progressive one, characterized by demyelination, followed by neuronal loss and motor function loss. An additional example is rheumatoid arthritis ("RA"). RA is believed to be an autoimmune disease arthritis ("RA"). RA is believed to be an autoimmune disease which involves chronic inflammation of the synovial joints and infiltration by activated T cells, macrophages and plasma cells. This inflamation is thought to lead to a progressive destruction of the articular cartilage. It is the most severe form of joint disease. The nature of the autoantigen(s) attacked in rheumatoid arthritis is poorly understood, but collagen type II is a candidate.

Like autoimmune diseases, rejection of transplanted tissue also involves a hyper-response by the immune system to an antigen. This is manifested as graft rejection in the case of organ transplantation (host-versus-graft disease, or HVGD). Another manifestation of pathological immune reactivity is graft-versus-host disease (GVHD) that occurs in approximately 30% of bone marrow recipients. Up to half of those patients who develop GVHD may succumb to this process. This high morbidity and mortality has led to continuous interest in the possibility of controlling or preventing GVHD.

There are two forms of GVHD, acute and chronic. Acute GVHD develops within the first 3 months after bone marrow transplantation and features disorders of skin, liver and gastrointestinal tract. Chronic GVHD is a multiorgan autoimmune-like disease, emerging from 3 months up to 3 years post-transplantation and shares features common to naturally occurring autoimmune disorders, like systemic lupus erythematosus (SLE) and scleroderma.

Studies on the effect of Copolymer 1 on various processes involved in the pathological course of immune rejection showed that Copolymer 1 inhibited T cell proliferation in response to host cell (Aharoni et al., Immunology Letters 58(2):79-87, 1997). Copolymer 1 treatment completely abolished cytotoxic activity toward grafts, prevented the pro-GVHD IL-2 and IFN-γ cytokine secretion, and induced beneficial Th2 anti-inflammatory response. In view of these cumulative data, Copolymer 1 is a candidate drug for the prevention of HVGD and GVHD in humans. See WO 96/32119 and U.S. Patent No. 5,858,964.
Copolymer 1 has been suggested as a potential therapeutic agent for multiple sclerosis (Eur. J. Immunol. [1971] 1:242; and J. Neurol. Sci. [1977] 31:433; K.P. Johnson, *1 Neurology* 65-70 (1995); N. Engl. J. Med. [1987] 317: 408) and other immune system conditions, such as immune diseases and delayed-type hyper-sensitivity conditions (WO 00/05250). This drug is a synthetic polypeptide functionally crossreactive with myelin basic protein (MBP). MBP is a natural component of the myelin sheath.

Copolymer 1 has been shown to suppress experimental allergic encephalomyelitis (EAE) induced by various encephalitogens, including mouse spinal cord homogenate (MSCH). MSCH encompasses all myelin antigens, such as MBP (Sela M. et al., 88 *Bull. Inst. Pasteur* 303-314 (1990), proteolipid protein (PLP) (Teitelbaum, D. et al., *J. Neurcimmunol.* (1996) 64: 209-217) and myelin oligodendrocyte glycoprotein (MOG) (Ben-Nun A et al., 243 J. Neurol. (Suppl 1) S14-S22 (1996)) in a variety of species. EAE is an accepted model for multiple sclerosis.

It has also been demonstrated that Copolymer 1 is active when injected subcutaneously, intra-peritoneally, intravenously or intramuscularly (D. Teitelbaum et al., *Eur. J. Immunol.* (1971) 1:242-248; D. Teitelbaum et al., *Eur. J. Immunol.* (1973) 3:273-279). For instance, in phase III clinical trials, daily subcutaneous injections of Copolymer 1 were found to slow progression of disability and reduce the relapse rate in exacerbating-remitting -multiple sclerosis (K.P. Johnson, *l Neurology* 65-70 (1995); N. Engl. J. Med. [1987] 317: 408).

Currently, all approved treatments of multiple sclerosis involve subcutaneous injection of the active substance. Frequently observed injection-site reactions include irritation, hypersensitivity, inflammation and pain and even necrosis (in the case of at least one interferon β 1-b treatment) and a low level of patient compliance. Therefore, an alternative method of administration is desirable. Thus, in order to effectively treat chronic diseases such as autoimmune diseases, oral, nasal or pulmonary formulations and methods for producing such formulations are necessary.

One way to overcome the difficulties in subcutaneous injection of drugs is to create a form that can be taken orally. EP Patent 359,783 discloses the treatment of autoimmune disease by oral administration of autoantigens. In addition, it describes the oral administration of MBP for the treatment of multiple sclerosis. Oral administration of an autoantigen has been termed "oral tolerance".

PCT International Application Nos. WO 91/12816, WO 91/08760, and WO 92/06704 all depict the treatment of autoimmune diseases other than multiple sclerosis using the "oral tolerance" method with a variety of autoantigens. However, none of these references disclose the treatment of multiple sclerosis or other autoimmune diseases by the oral administration of a non-autoantigen such as Copolymer 1.

The invention also encompasses another avenue of administration that avoids the problems inherent in subcutaneous injection. This avenue is to produce a form of the drug that can be administered nasally.

The disclosed invention provides pharmaceutical compositions useful for treating autoimmune diseases in a mammal comprising as an active ingredient a therapeutically effective amount of Copolymer 1, an amount of microcrystalline cellulose in excess of 50% by the weight of the composition, and an enteric coating. Processes for the manufacture of such compositions are also disclosed.
- **Figure 1**: shows the results of trials ascertaining prevention and treatment of EAE in Rhesus monkeys fed with Copolymer 1 (glatiramer acetate - "GA") enteric coated capsules.
- **Figure 2**: shows prevention of EAE in Rhesus monkeys fed with Copolymer 1 enteric coated capsules.
- **Figure 3**: shows the drug release profile of Copolymer 1 enteric-coated tablets.
- **Figure 4**: shows the effect of daily oral Gavage treatment with Copolymer 1 solution on rat EAE.
- **Figure 5**: shows the results of treatment of chronic-relapsing (CR)-EAE in Biozzi mice with oral Copolymer 1 solution.
- **Figure 6**: shows the results of treatment of EAE treatment in Cynamologus monkeys by oral administration of Copolymer 1 in enteric-coated tablets.

This invention provides a pharmaceutical composition in solid form comprising as an active ingredient a therapeutically effective amount of Copolymer 1, an amount of microcrystalline cellulose in excess of 50% by the weight of the composition, and an enteric coating.

The amount of microcrystalline cellulose is at least 50 % by weight, preferably from 60% to 90% by weight, more preferably at least 70 % by weight, most preferably from 70% to 80% by weight.

The microcrystalline cellulose may have a moisture content of up to 5.0% or a moisture content of up to 1.5%.

The pharmaceutical composition may further comprise a disintegrant. The disintegrant may be selected from the group consisting of kaolin, starch, powdered sugar, sodium starch glycolate, crosscarmelose sodium, carboxymethyl cellulose, microcrystalline cellulose and sodium alginate. Preferably, the disintegrant is a pregelatinized starch. The starch may have a moisture content of up to 14%, preferably a moisture content of up to 12%, more preferably a moisture content of up to 7%, most preferably a moisture content of up to 5%.

The pharmaceutical composition may further comprise a lubricant. The lubricant may be selected from the group consisting of talc, sodium stearyl fumarate, magnesium stearate, calcium stearate, hydrogenated castor oil, hydrogenated soybean oil, and polyethylene glycol. Preferably, the lubricant is magnesium stearate.

The pharmaceutical composition may further comprise a protease inhibitor.

The pharmaceutical composition comprises an enteric coating. The enteric coating may be methacrylic ester copolymer, cellulose acetate phthalate (CAP), hydroxypropyl methyl cellulose phthalate (HPMCP), carboxymethyl ethyl cellulose (CMEC), amino-alkylmethacrylate copolymer. Preferably, the enteric coating is methacrylic acid copolymer.

The pharmaceutical composition may further comprise a film coating under the enteric coating. The film coating may be selected from the group consisting of hydroxy propyl methyl cellulose (HPMC) and poly vinyl alcohol (PVA).

The pharmaceutical composition is in solid form. The solid form may be selected from the group consisting of a tablet, a hard gelatin capsule, a pellet and a particulate formulation.

It may be a tablet and the effective amount of Copolymer 1 is from about 0.1 mg to about 300 mg, preferably from about 5 mg to about 100 mg. In another embodiment, the effective amount of Copolymer 1 is from about 5 mg to about 50 mg. In a preferred composition, the effective amount of Copolymer 1 is about 5 mg. In another preferred embodiment, the effective amount of Copolymer 1 is about 10 mg. In a further preferred composition, the effective amount of Copolymer 1 is about 50 mg. In one embodiment, the effective amount of Copolymer 1 is about 0.01 mg/kg to about 2 mg/kg. In a preferred composition, the effective amount of Copolymer 1 is about 0.05 mg/kg to about 1 mg/kg.

In a preferred embodiment, the pharmaceutical composition in solid form comprises as an active ingredient a therapeutically effective amount of Copolymer 1, 70%-80% by weight of microcrystalline cellulose, an enteric coating, and either 5 mg or 50 mg of Copolymer 1.

The pharmaceutical composition may further comprise a pharmaceutically acceptable carrier suitable for application to mucosal linings, so as to thereby form a composition suitable for application to the mucosal linings of a subject. Preferably, the carrier is chitosan.

The pharmaceutical composition may further comprise a pharmaceutically effective amount of an anti-microbial preservative. The anti-microbial preservative may be selected from the group consisting of sodium benzoate, methyl paraben, benzalkonium chloride, and propyl paraben.

According to this invention, the pharmaceutical composition may be in dry powder form.

The mucosal linings to which the pharmaceutical composition may be administered may be bronchi-associated lymphoid tissue.

The pharmaceutical composition may be formulated for either oral administration, buccal administration, nasal administration, or pulmonary administration.

Also disclosed is a process for manufacturing the pharmaceutical composition, wherein the process comprises, a) milling the Copolymer 1, b) dry mixing and/or granulating the milled Copolymer 1 with at least 50% by weight of microcrystalline cellulose, and c) applying an enteric coating.

The process may further comprise applying a film coating. The enteric coating may be applied using a rotating pan system.

Also disclosed is the use of the pharmaceutical composition according to this invention for the manufacture of a medicament for treating an autoimmune disease in a mammal. The autoimmune disease is selected from the group consisting of an arthritic condition, autoimmune hemolytic anemia, autoimmune oophoritis, autoimmune uveoretinitis, Crohn's disease, chronic immune thrombocytopenic purpura, colitis, contact sensitivity disease, Graves disease, Guillain-Barre's syndrome, Hashimoto's disease, idiopathic myxedema, myasthenia gravis, psoriasis, pemphigus vulgaris, rheumatoid arthritis, GVHD or HVGD.

Preferably, the autoimmune disease is multiple sclerosis.

Thus, this invention provides a pharmaceutical composition useful in the treatment of autoimmune diseases by oral administration of Copolymer 1. When Copolymer 1 is introduced orally, it may be mixed with other food forms and consumed in solid, semi-solid, suspension or emulsion form; and it may be mixed with pharmaceutically acceptable carriers, including water, suspending agents, emulsifying agents, adjuvants, flavor enhancers and the like. The oral composition is enterically-coated. Use of enteric coatings are well known in the art. Commonly known enteric coatings include Eudragit S and Eudragit L (K. Lehman, Acrylic Coatings in Controlled Release Tablet Manufacture, Manufacturing Chemist and Aerosol News, p. 39 (June 1973); K. Lehman, Programmed Drug Release from Oral Program Forms, Pharma. Int., vol. ISS 3 1971, p. 34-41; Handbook' of Pharmaceutical Excipients, 2^{nd} ed.).

An additional object of the present invention is to provide forms of Copolymer 1 that can be administered nasally to treat autoimmune conditions. For instance, Copolymer 1 may be administered as dry powder or metered dose of solution by inhalation, or nose-drops and nasal sprays, using appropriate formulations and metered dosing units. These formulations are intended to deliver Copolymer 1 to either (i) mucosal linings of the lungs and associated airways or (ii) mucosal linings of the nasal cavities.

As contemplated, Copolymer 1 is brought into contact with those lymphoid tissues in the mucosal linings which are believed to be a primary source of immune system sensitization. These mucosal linings may be found (though not necessarily exclusively) in the sinuses, trachea, bronchial passages (where they are known as the BALT or bronchi-associated lymphoid tissues) and gastrointestinal linings (known as GALT or gut-associated lymphoid tissues). Thus, the administration of Copolymer 1 is understood to include methods wherein Copolymer 1 is introduced into the body by way of ingestion or inhalation. For example, Copolymer 1 may be administered by way of the mouth through feeding, through a stomach tube, by inhalation into the bronchial passages or by nasal inhalation.

The composition contemplated by the subject invention may be administered either as a simple oral solution, as an emulsion or suspension formulation, as a solid oral dosage form (capsule or tablet), or even as a soft gelatin capsule. The present invention contemplates immediate release dosage forms and modified release dosage forms (including particulates, coated granules and pellets, emulsions, microemulsions and encapsulation in microspheres and nanespheres).

Enteric-coated dosage forms include the enteric-coated tablet, soft and hard gelatin capsule, pellet, particle and microparticle formulations. For instance, one may employ Eudragit (methacrylic acid copolymer) or Opadry (hydroxy propyl methyl cullulose ("HPMC") or poly vinyl alcohol ("PVA")) coatings, designed to target release by pH control in the stomach or in the gut, in the duodenum, jejunum, ileum or colon. Control of release may also be achieved through matrix erosion or time release formulations. Inactive ingredients can include lactose, microcrystalline cellulose, mannitol, PVP, starch, sodium starch glycolate, stearic acid, talc, hydrogenated triglycerides, polylactic acid and polyglycolic acid copolymers, or other ingredients intended specifically to enhance gut absorption.

Solid oral dosage form manufacturing processes can include direct compression and tableting, dry granulation, wet granulation, particulate and pellet manufacture by extrusion, spheronisation and melt granulation. The bulk drug substance may be mixed or milled prior to blending with other excipients, or co-dissolved and sprayed in a solution in a wet granulation process. Spray drying may also be an appropriate method to achieve therapeutically important particulate formulations with or without an inactive ingredient (such as lactose). The amount of active ingredient within the composition can be in the range of 0.01 mg to 1000 mg, while the range 0.1 mg to 300 mg is likely to be optimal.

Autoimmune diseases contemplated by the present invention include either cell-mediated disease (e.g., T cell) or antibody-mediated (e.g., B cell) disorders. Such disorders can be *inter alia,* arthritic conditions, demyelinating diseases and inflammatory diseases. For example, autoimmune diseases which can be treated by the present compositions include multiple sclerosis, arthritic conditions, autoimmune hemolytic anemia, autoimmune oophoritis, autoimmune uveoretinitis, Crohn's disease, chronic immune thrombocytopenic purpura, colitis, contact sensitivity disease, Graves disease, Guillain-Barre's syndrome, Hashimoto's disease, idiopathic myxedema,, myasthenia gravis, psoriasis, pemphigus vulgaris, rheumatoid arthritis, and GVHD or HVGD. The present compositions can be used to treat one or more of these diseases.

The phrase, "arthritic condition", as used herein is a condition wherein at least one symptom of rheumatoid arthritis is observed in at least one joint of a mammal, for example in a shoulder, knee, hip, backbone or digit. Examples of arthritic conditions include "polyarthritis", which is an arthritic condition that affects more than a single joint; "juvenile arthritis", an arthritic condition of-humans under the age of 21; and Felty's syndrome, which can include the symptoms of neutropenia, splenomegaly, weight loss, anemia, lymphadenopathy, and pigment spots on the skin.

It is to be understood and expected that variations in the principles of invention herein disclosed may be made by one skilled in the art and it is intended that such modifications are to be included within the scope of the present invention.

### Experimental Details

### EXAMPLE 1: PRODUCTION OF ENTERIC-COATED CAPSULES AND TABLETS

### Experiment 1A

Initial pre-clinical and clinical studies were performed using enteric-coated hard gelatin capsule formulations. All formulations were tested to meet (i) assay and impurities specifications, (ii) quality specifications for Copolymer 1, (iii) USP requirements for uniformity of dosage units by content uniformity and (iv) USP requirements for drug release for delayed release (enteric-coated) articles. Together, these tests were intended to ensure that each lot meets the necessary requirements for identity, strength, quality and purity.

Experimental lots of enteric-coated hard gelatin capsules were prepared for initial evaluation in monkeys suffering from EAE, manufactured using co-lyophilized Copolymer 1 and mannitol, sieved and hand-filled into hard gelatin capsules. The capsules were film-coated with Opadry (white) (commercial grades of hydroxy propyl methyl cellulose (HPMC) coating formulations marketed by Colorcon® (UK)) and then enteric-coated using methacrylic acid copolymer NF (Eudragit L-30 D-55, a commercial grade of methacrylic acid copolymer, available as a stabilized 30% aqueous dispersion, manufactured by Rohm® (Germany)) using a Würster fluid bed dryer (Accelacota 10).

The methacrylic acid copolymer used was Eudragit L 30 D-55, which is an aqueous dispersion of a methacrylic acid and acrylic acid ethyl ester. The ratio of free carbonyl groups to ester groups is 1:1. The films dissolve above pH 5.5 forming salts with alkali, thus affording coatings which are insoluble in gastric media, but soluble in the small intestine.

**Table 1** lists the inactive ingredients employed in the hard gelatin capsules and the purpose of each component. Capsules containing 1 mg and 20 mg of active ingredient were developed.

**TABLE 1: Excipients in Hard Gelatin Capsule Formulations**

| **Inactive Ingredient** | **Pharmaceutical Function** |
|---|---|
| *Powder-Fill* | |
| Lactose Monohydrate NF | Filler |
| Silicon Dioxide NF | Glidant |
| Pregelatinized Starch NF | Disintegrant |
| Magnesium Stearate NF | Lubricant |

| *Film-Coating Suspension* | |
|---|---|
| Opadry YS-1-7006 (clear) | Film coating |
| Purified Water USP | Coating suspension solvent* |

| *Enteric-Coating Suspension* | |
|---|---|
| Methacrylic Acid Copolymer NF (Eudragit L-30 D-55) | Enteric-coating |
| Talc USP | Glidant |
| Triethyl Citrate NF | Plasticizer |
| Purified Water USP | Coating suspension solvent* |

| | |
|---|---|
| *Coating solvent evaporated to dryness | |

### i) Composition

**TABLE 2**

| Components | Composition (mg/capsule) | |
|---|---|---|
| Strength | 1 mg | 20 mg |
| *Powder Fill* | | |
| Copolymer 1 | 1.0 | 20.0 |
| Mannitol USP | 48.8 | 17.5 |
| | | |

| *Seal coat* | | |
|---|---|---|
| Opadry (S-OY-7399) white | 32.7 | 32.7 |
| Purified Water USP | * | * |
| | | |

| *Enteric Coat* | | |
|---|---|---|
| Merthacrylic acid copolymer NF (Eudragit L30 D55) | 20.3 | 20.3 |
| Talc USP | 10.3 | 10.3 |
| Triethyl citrate NF | 2.1 | 2.1 |
| Purified Water USP | * | * |

| | | |
|---|---|---|
| * Processing solvent evaporated to dryness. | | |

### (ii) Manufacturing Process

1. Compounding of solution of 1.2 L of Copolymer 1 and mannitol in water
2. Lyophilization in bulk in Edward's lyoflex 0.5 pilot lyophilizer
3. Sieving - mesh #20/mesh#50.
4. Capsule filing - semi-manual Feton capsule filing machine.
5. Film coating Opadry (S-OY-7399 white) in Würster fluid bed dryer.
6. Enteric coating (Eudragit L30 D55 spraying suspension) in Würster fluid bed dryer.

Rhesus monkeys were fed with the enteric coated capsules. 5 feedings before disease induction and 5 feedings after disease induction, on alternate days. Experimental allergic Encephalomyelitis (EAE) was induced by injecting 8 mg myelin basic protein (MBP) in complete Freund's adjuvant (CFA) intradermally into the leg. EAE symptoms were evaluated on the following scale:

### Evaluation of EAE clinical signs:

| Score | Clinical State |
|---|---|
| 0 | Normal neurological exam and appearance |
| 1 | Weight loss, anorexia, yawning, slow responses to stimuli, irritability or lethargy |
| 2 | Mild neurological signs, indifference, drooling, clumsiness using limbs, ataxia, tremor, altered cry and disordered gaze |
| 3 | Moderate neurological signs, blindness (pupils do not react to light), akinesia, leg weakness or paralysis |
| 4 | Severe neurological signs, semicoma, coma, quadriplegia. When the animal reached a score of 4 it was sacrificed for humane reasons |
| 5 | Death |

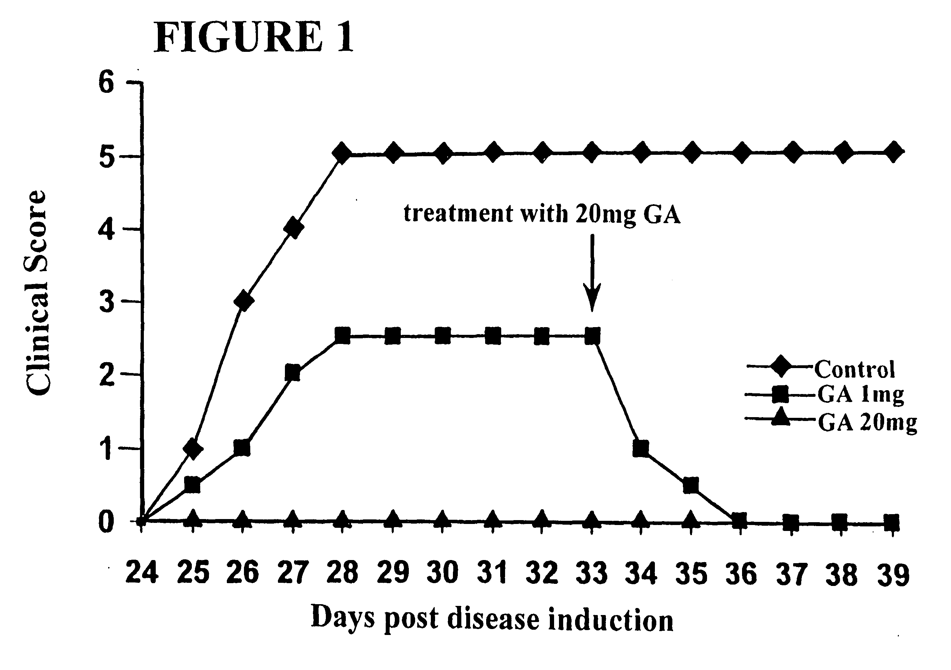

As can be seen in Fig. 1, control animals developed EAE on day 25 post disease induction and died within 4 days from the disease.

Animals that were fed with 20 mg Copoiymer 1 in enteric coated capsules did not develop any signs of disease till day 60, when it was sacrificed for histology. One animal per group was used.

The animal that was treated with 1 mg Copolymer 1 in enteric coated capsules developed some signs of disease (score 2⁺), but upon treatment with 20 mg capsules healed completely.

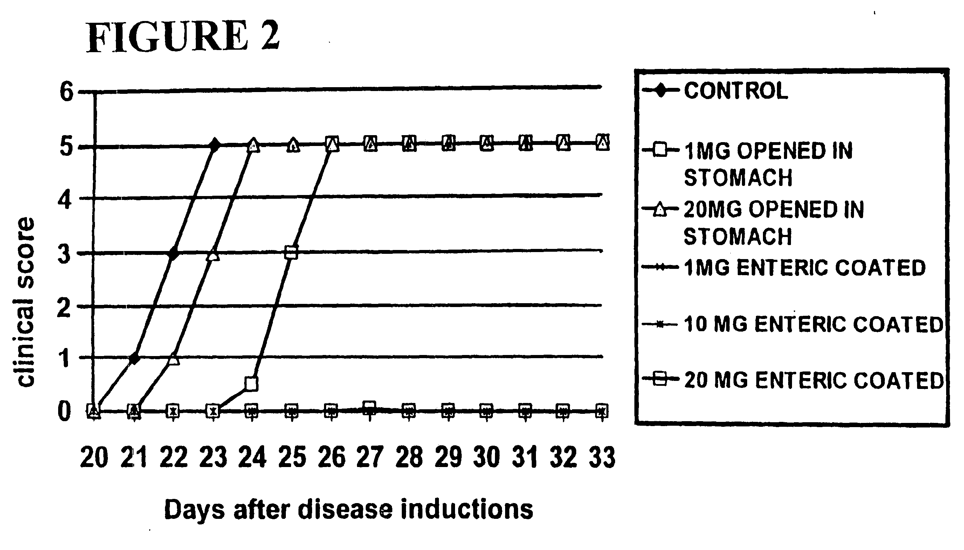

In a second study (Fig. 2), all monkeys treated with Copolymer 1 enteric coated capsules (1, 10, and 20 mg) were fully protected from EAE while monkeys treated with Copolymer 1 in capsules which were not gastro-protective (opened in the stomach) or control capsule died from the disease, indicating the importance of the enteric coating in these dose levels (1 to 20 mg.).

### Experiment 1B

The procedure of Experiment 1A was adapted to give a stable product under refrigerated conditions that (i) can be manufactured using standard industrial pharmaceutical processes and (ii) meets pre-defined quality specifications appropriate for initial clinical evaluation. Here, the previous experiment's process of manufacturing the powder-fill was replaced by a dry granulation process.

### (i) Composition

**TABLE 3**

| Components | composition mg/capsule | | |
|---|---|---|---|
| Strength | Placebo | 20 mg | 100 mg |
| *Powder Fill* | | | |
| Copolymer 1 | 0.0 | 20.0 | 100.0 |
| Lactose monohydrate NF | 322.7 | 36.6 | 183.2 |
| Pregelatinized starch NF | 37.3 | 6.7 | 33.3 |
| Silicon Dioxide NF | 9.3 | 1.7 | 8.3 |
| Magnesium Stearate NF | 3.7 | 0.7 | 3.3 |

| *Seal Coat* | | | |
|---|---|---|---|
| Opadry (YS-1-7006 clear) | 29.0 | 29.0 | 29.0 |
| Purified Water USP | . | . | . |

| *Enteric Coat* | | | |
|---|---|---|---|
| Methacrylic acid copolymer NF (Eudragit L30 D55) | 50.6 | 50.6 | 50.6 |
| Talc USP | 25.4 | 25.4 | 25.4 |
| Triethyl citrate NF | 8.0 | 8.0 | 8.0 |
| Purified Water USP | . | . | . |

| | | | |
|---|---|---|---|
| * Processing solvent evaporated to dryness. | | | |

### (ii) Manufacturing process (6kg)

1. Milling of Copolymer 1 - (fitzmill milling machine)
2. Blending with excipients - Y-cone 15 blender
3. Compression of slugs (20mm, 1g) - Killian tableting machine
4. Granulation of slugs - Frevitt granulator, 0.8 mm net
5. Blending with excipients - Y-cone 15 blender
6. Capsule filling - Bosch 400 encapsulator.
7. Coating (Accelacota-10 rotating pan machine).

The clinical formulation was administered to patients suffering from multiple sclerosis (MS) in the framework of the Phase I study. It was demonstrated that Copolymer 1 (20, 100 and 300 mg.) in enteric coated capsules was tolerated and safe in MS patients.

In addition, oral administration of Copolymer 1 in enteric coated capsules modified the cytokine profile in multiple sclerosis patients (increase of IL-10 and decrease in IL-2) indicating a possible clinical effect of this formulation in MS patients.

### Experiment 1C

Enteric-coated tablets (7.0 mm, round, 20 mg active ingredient) were chosen for further toxicological evaluation in monkeys. To create a stable product at controlled room temperature, Experiment 1B's procedure was modified. The lactose monohydrate filler used in the capsule formulations was replaced with microcrystalline cellulose NF (Avicel PH 102) due to the observation of long-term incompatibility with the active ingredient (a Maillard reaction occurred under accelerated storage conditions that resulted in yellowing of the granulate inside the capsule). Sodium starch glycolate NF was employed as the disintegrant; the slightly more rapid drug release brought about by sodium starch glycolate NF assisted in the toxicological evaluation in monkeys, where the gut is shorter than in humans.

A different opadry grade (Y-1-7000H) was used, even though this is also hydroxy propyl methyl cellulose ("HPMC")-based.

A corresponding placebo formulation was also developed.

The microcrystalline cellulose used was Avicel® PH 102, although Avicel® PH 101, and Avicel® PH 112, all manufactured by FMC Corporation, could be used. Microcrystalline cellulose fillers were employed in the experiments for tablet formulation. The three different kinds of microcrystalline cellulose binders mentioned differ in moisture content. Avicel® PH 101 and Avicel® PH 102 are both high moisture content microcrystalline cellulose binders having less than or equal to 5% moisture content. Avicel® PH 112 is a low moisture content microcrystalline cellulose binder having less than or equal to 1.5% moisture content. The mean particle size of Avicel ® ranges from 20 to 200 microns. The mean particle size of Avicel® PH 102 is 100 microns.

### (i) Composition

**TABLE 4**

| Ingredients | Amount (mg)/Tablet | |
|---|---|---|
| | 0 mg | 20 mg |
| *Tablet Core* | | |
| Copolymer 1 | 0.0 | 20.0 |
| Microcrystalline cellulose NF | 105.2 | 71.5 |
| Silicon dioxide NF | 2.9 | 2.5 |
| Sodium starch glycolate NF | 5.8 | 5.0 |
| Magnesium stearate NF | 1.1 | 1.0 |
| | | |

| *Enteric Coat* | | |
|---|---|---|
| Methacrylic acid copolymer NF (Eudragit L-30 D-55) | 9.4 | 9.4 |
| Talc USP | 4.7 | 4.7 |
| Triethyl citrate NF | 0.9 | 0.9 |
| Purified water USP | * | * |
| Total coating weight per tablet | 15.0 | 15.0 |
| Final coated tablet weight | 130.0 | 115.0 |

| | | |
|---|---|---|
| * Processing solvent evaporated to dryness. | | |

### (ii) Manufacturing process (6 kg)

1. Milling of Copolymer 1 - (fitzmill milling machine)
2. Blending with excipients - Y-cone 15 blender
3. Compression of slugs (20 mm, 1g) - Killian tableting machine
4. Granulation of slugs - Frevitt granulator, 0.8 mm net
5. Blending with excipients - Y-cone 15 blender
6. Tableting - Killian tableting machine.
7. Coating (Accelacota-10 rotating pan machine).

### Experiment 1D

Experiment 1C's procedure was adapted to create similar formulations of enteric-coated tablets for clinical evaluation in humans suffering from multiple sclerosis. Pregelatinized starch was employed as the disintegrant. Tablet shape and size were selected as appropriate for enteric administration and found to be suitable for the intended pharmaceutical manufacturing processes, including coating. The 5 mg active ingredient tablets (and corresponding placebos) were manufactured in the same shape and size as in Experiment 1C. The size and shape of the 50 mg active ingredient tablets (and corresponding placebos) was changed into 14.7 mm x 8.1 mm oval-shaped tablets appropriate for enteric administration and the intended pharmaceutical manufacturing processes, including coating.

Scale-up and process qualification studies were completed for 52 kg lots of tablet cores for both the 5 mg and 50 mg active ingredient. The tablet cores' composition is presented below.
(i) Composition of Tablet Cores

| | | | | |
|---|---|---|---|---|
| TABLE 5 | | | | |

| **Ingredients** | **amounts of active ingredient (mg/tablet)** | | | |
|---|---|---|---|---|
| | **0 mg** | **0 mg** | **5 mg** | **50 mg** |
| *Tablet Core* | | | | |
| Copolymer 1 | 0.0 | 0.0 | 5.0 | 50.0 |
| Microcrystalline cellulose NF (Avicel PH 102) | 105.2 | 366.0 | 86.5 | 316.0 |
| Silicon dioxide NF | 2.9 | 10.0 | 2.5 | 10.0 |
| Pregelatinized starch NF | 5.8 | 20.0 | 5.0 | 20.0 |
| Magnesium stearate NF | 1.1 | 4.0 | 1.0 | 4.0 |
| Total | 115.0 | 400.0 | 100.0 400.0 | |

| *Enteric Coat* | | | | |
|---|---|---|---|---|
| Methlacrylic acid copolymer NF (Eudragit L-30 D-55) | 9.4 | 21.9 | 9.4 | 21.9 |
| Talc USP' | 4.7 | 11.0 | 4.7 | 11.0 |
| Triethyl citrate NF | 0.9 | 2.1 | 0.9 | 2.1 |
| Purified water USP | * | * | * | * |
| Total coating weight per tablet | 15.0 | 35.0 | 15.0 | 35.0 |
| Final coated tablet weight | 130.0 | 435.0 | 115.0 | 435.0 |
| * Processing solvent evaporated to dryness. | | | | |

(ii) Manufacturing process (52 kg)
1. Milling of Copolymer 1 - (fitzmill milling machine)
2. Blending with excipients - Y-cone 120 blender
3. Compression of slugs (20 mm, 1g) - Killian tableting machine
4. Granulation of slugs - Frevitt granulator, 0.8 mm net
5. Blending with excipients - Y-cone 120 blender
6. Tableting - Killian tableting machine.
7. Coating sub-lots (Accelacota-10 rotating pan machine).
   Accelerated stability testing (at 40°C/75% Relative Humidity) of lots with

(i) different film-coating Opadry formulations; and
(ii) different slug hardness values indicated that optimal formulations can be achieved by carefully controlling slug hardness and using L30 D55 Eudragit with or without Opadry film coat (or any other hydroxy propyl methyl cellulose (HPMC) formulation).

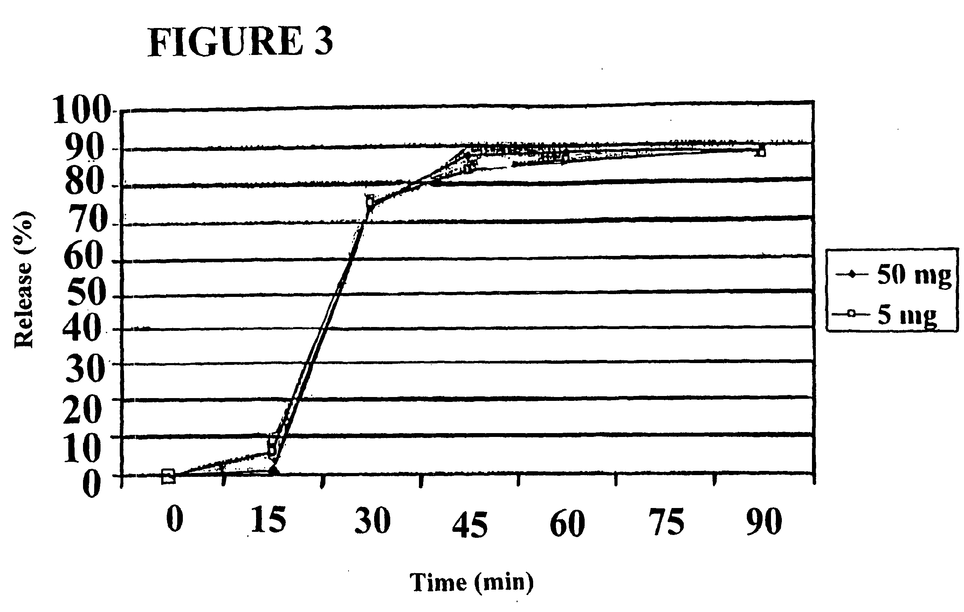

The dissolution profiles of the 5 mg and the 50 mg tablets were compared and found to be matching despite the four-fold difference in tablet weight. The dissolution profiles (in buffer at pH 6.8) are shown in Figure 3.

### EXAMPLE 2: ORAL ADMINISTRATION OF COPOLYMER 1 SOLUTION

### Example 2A:

### Gavage Administration of Copolymer 1 in Solution to Rats

EAE was induced in Lewis rats by the injection of guinea pig spinal cord homogenate in CFA into the hind legs.

For EAE suppression, rats were fed with Copolymer 1 solution (0.1-10 mg/kg.) every day, starting from the day of EAE induction, up to day 24 (test termination).

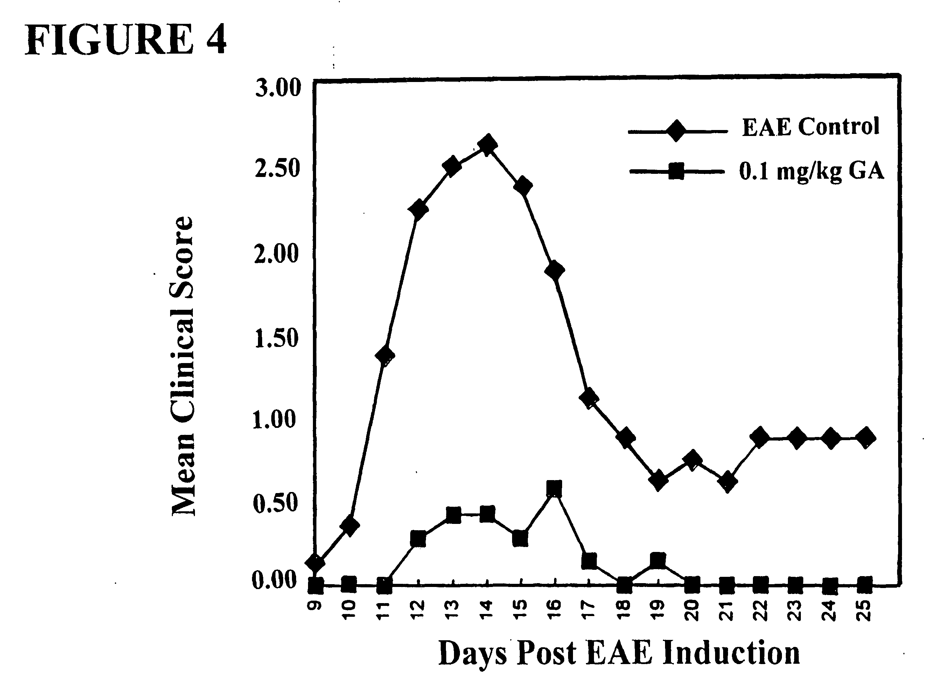

Copolymer 1 inhibited EAE at all tested doses, with bimodal dose response pattern. The most effective doses were the lowest dose 0.1 mg/kg (see Fig. 4) and the highest dose of 10 mg/kg.

In all groups, Copolymer 1 delayed disease onset, reduced the % of sick rats, the severity of disease (as expressed by the mean score and the mean maximal score) and the disease duration.

### Example 2B: Suppression of Chronic - Relapsing EAE in Biozzi Mice by Oral Adminstration of Copolymer 1 in Solution

The chronic - relapsing EAE model represents MS better due to its relapsing nature. It enables the demonstration of drug effect on a disease which is on-going, similar to the situation of treating the human disease, which is on-going.

Chronic - relapsing EAE was induced in female Biozzi mice by the injection of mouse spinal cord homogenate (MSCH) in CFA, following by re-injection of the encephalitogen one week later, in the same manner.

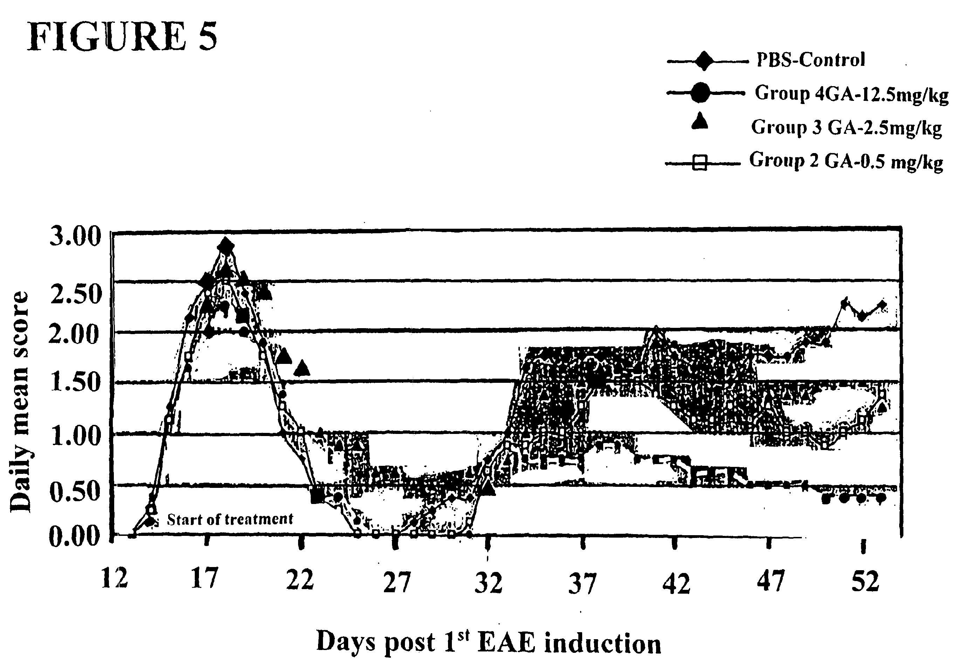

On day 16 when the mice were already sick (see Fig. 5), they were randomized into 4 groups and treated daily by either phosphate buffer saline (PBS) as control or with Copolymer 1 solution (0.5 mg/kg/day, 2.5 mg/kg/day and 12.5 mg/kg/day).

As can be seen in Fig. 5, treatment with Copolymer 1 reduced the EAE clinical symptoms of the second relapse in a dose-dependent manner. The most effective dose was 12.5 mg/kg. The suppressive effect was demonstrated in all parameters checked: the incidence of mice suffering from second relapse, the severity of symptoms and the duration of the relapse.

### EXAMPLE 3: ORAL ADMINISTRATION OF COPOLYMER 1 TABLETS

### Example 3A: Induction of Relapsing-Remitting EAE in Cynamologus Monkeys

A relapsing-remitting form of EAE was induced by the injection of emulsified MBP in CFA containing 3mg/ml of Mycobacterium Tuberculosis (MT), intradermally into right & left footpads.

The daily dosages employed are preserted in Table 6 below. Observation was initiated on day 8. Local reaction was observed at the site of injection, an ulcer/or bleeding ulcer caused intermittent use of the foot and /or flexion of the toes. Neurological signs appeared on day 14 following induction, as shown in Table 6.

| | | | | | | | |
|---|---|---|---|---|---|---|---|
| Table 6: Daily Clinical Score | | | | | | | |

| Monkey I.D. | Dose | DAYS | | | | | |
|---|---|---|---|---|---|---|---|
| | | 14 | 15 | 16 | 17 | 18 | 19-32 |
| Female Z-920 | 2.5 mg | 3 | 3 | 5 | | | |
| Female Z-929 | 5 mg | 0 | 0 | 3 | 4* | | |
| Female Z-485 | 10 mg | 1 | 2 | 3 | 5 | | |
| Male Z-523 | 2.5 mg | 0 | 2 | 3 | 3/4 | 4* | |
| Male Z-1669 | 5 mg | 0 | 0 | 0 | 0 | 0 | 0 |
| Male Z-701 | 10 mg | 0 | 0 | 0 | 0 | 0 | 0 |
| * euthanized | | | | | | | |

Table 6 demonstrates that the appearance of EAE was acute, (grade 3). Two of the females died within 48 hours of onset of signs of EAE, whereas the third female was euthanized subsequent to the development of grade 4 signs (coma).

Only one male developed signs of EAE and was euthanized after it reached grade 4. The two males which did not develop signs of EAE were euthanized after 32 days.

Thus, the dose injected for the induction of EAE does not relate to the time interval between injection and appearance of signs of EAE, or the severity of signs. The females were the first to develop the signs of EAE, and the first to die. Only a third of the males developed EAE.

### Example 3B: Treatment of EAE in Cynamologus Monkeys by Oral Administration of Copolymer 1 in Enteric-Coated Tablets

Twelve Cynomologus monkeys weighing 3 to 4 kg at the age of approximately four years were randomly divided into four treatment groups. For 10 days on every alternate day (total of 5 treatments), each monkey was introduced with the tablets using tip coated forceps beyond the radix of the tongue, after which swallowing was assured. The groups were treated as follows:
Group #1 (placebo control): four placebo tablets
Group #2 (5 mg Copolymer 1 per treatment): one tablet of 5 mg Copolymer 1 and 3 placebo tablets
Group #3 (10 mg Copolymer 1 per treatment)': two tablets of 5 mg Copolymer 1 and 2 placebo tablets
Group #4 (20 mg Copolymer 1 per treatment): four tablets of 5 mg Copolymer 1.

Subsequently, following the procedure of Example 3A, EAE was induced in all monkeys by single injection with 2.5 mg of MBP per monkey. Simultaneously, each group began daily treatment with the same dose of Copolymer 1 as they received prior to the induction of EAE for 30 days.

In the first study, Cop-1 treatment continued until day 52. In the second study, Cop-1 treatment continued until day 57. MBP was administered in a single administration. Cop-1 was administered continuously except for several weekends in the first study, and except for Saturdays in the second study.

The monkeys were examined every Sunday to Friday, and also not monitored on Saturdays, starting from day 8 after disease induction until day 52. At the end of this period, the monkeys were sacrificed and the tissue was harvested.

Local reaction was observed at the site of injection, such as an ulcer and/or bleeding ulcer, which caused intermittent use of the foot and/or flexion of the toes. EAE-related neurological signs appeared between day 12 and 20 following induction, as specified in Table 7 below.

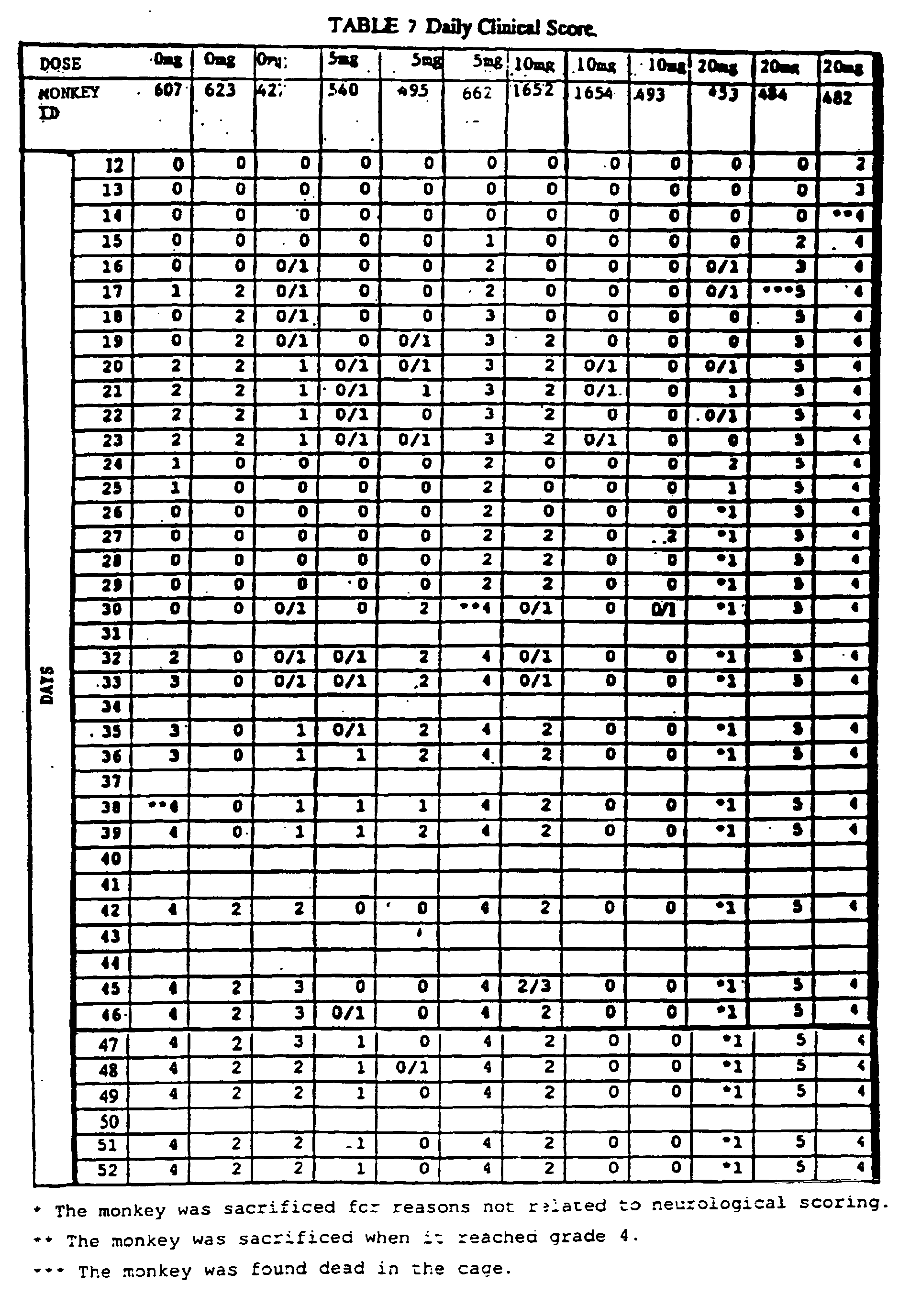

All three females in the placebo group showed signs of illness on days 17-23 followed by a remission period of a week. Female No. 607 had a second acute relapse on day 32 and was sacrificed on day 38 when score 4 was reached. Females No. 623 and 427 showed severe signs of illness in the last 10 days of follow up (scores 2 and 3).

In the 5 mg Copolymer 1 treatment group, Female No. 662 showed signs of illness on day 15 and by day 18, after losing her pupillary reflex, received score 3. On day 30, the animal was sacrificed after it reached score 4. The two other females, No. 540 and 495, had a similar sequence of symptoms, with mild signs on day 18/19 to day 23 (score 1) and a relapse between day 30/32 and day 39 (score 1-2). Female No. 540 had a third relapse between day 46 to day 52 (score 1).

In the group that was treated with 10 mg Copolymer 1, Female No. 1652 showed neurological symptoms from day 19 (score 2), with a short remission period between days 24-26 and 30-33(score 0/1) and survived to the end of the follow up period. Females No. 493 and 1654 showed irregular, very mild symptoms during the entire follow up period (score fluctuated between 0 and 0/1).

In the 20 mg Copolymer 1 treatment group, Females No. 482 and 484 showed a very acute onset of signs. Female No. 482 reached score 2 on day 12, 3 on day 13 and was euthanized on day 14 (score 4).

Female No. 484 registered score 2 on day 15, reached score 3 the following day and was found dead on day 17 (score 5). Female No. 453 had mild neurological symptoms, but because of suppurative infected inguinal lymph nodes and infected bleeding wounds of the foot pad, which caused the animal severe deterioration, the animal was sacrificed on day 26. Seven females survived to the end of the follow up period and were sacrificed on day 53. Inguinal lymph nodes and sections of the intestine were harvested for immunochemical staining.

The results of this example are summarized in Table 8 below.

**TABLE 8: Evaluation of Clinical Manifestations**

| Treatment Group | Incidence | Mean Score | Mean Maximal Score | No. Relapses* | Mean Disease Onset Day |
|---|---|---|---|---|---|
| Control | 3/3 | 1.27 | 3.00 | 3 (6) | 16.66 |
| 5 mg | 3/3 | 1.30 | 2.33 | 2 (5) | 18.33 |
| 10 mg | 1/3 | 0.49 | 1.33 | 1 (3) | 23 |
| 20 mg | 3/3 | 3.09 | 3.67 | 0 (3) | 14.33 |

In summary, the course of EAE was phasic, with recurrence of symptoms and remission periods. While the placebo group showed the chronic phasic pattern of the disease, leading to deterioration with time, the 20 mg Copolymer 1 treatment group had an acute onset of symptoms with a fulminate course of the disease and death. The group treated with 5 mg Copolymer 1 exhibited a pattern of disease similar to the placebo group with no obvious improvement. In the 10 mg Copolymer 1 group, two females showed irregular, very mild symptoms, while only one female showed more than very mild symptoms (score 2 with periods of remission to score 0/1). All three survived through the whole study. The difference among the placebo, 10 mg Copolymer 1 treatment group and 20 mg treatment Copolymer 1 group is significant (p < 0.0001). However, the difference between the placebo group and the 5 mg group is not significant (n.s.) (p = n.s.).

These results suggest that the most effective dosage is 10 mg Copolymer 1.

### Example 3C: Treatment of EAE in Cynamologus Monkeys by Oral Administration of Copolymer 1 in Enteric-Coated Tablets

The procedure of Example 3B was followed with the following exceptions - there was no 5 mg Copolymer 1 treatment group and the monkeys were observed until day 57 after disease induction.

As in Example 3B, observation began on day 8 and continued until the conclusion of the experiment, which, in this case, was day 57. Local reaction was observed at the site of injection, such as an ulcer and/or bleeding ulcer, which caused intermittent use of the foot and/or flexion of the toes. EAE-related neurological signs appeared between day 14 and 54 as specified in Table 9. Animals were sacrificed when they reached score 4.

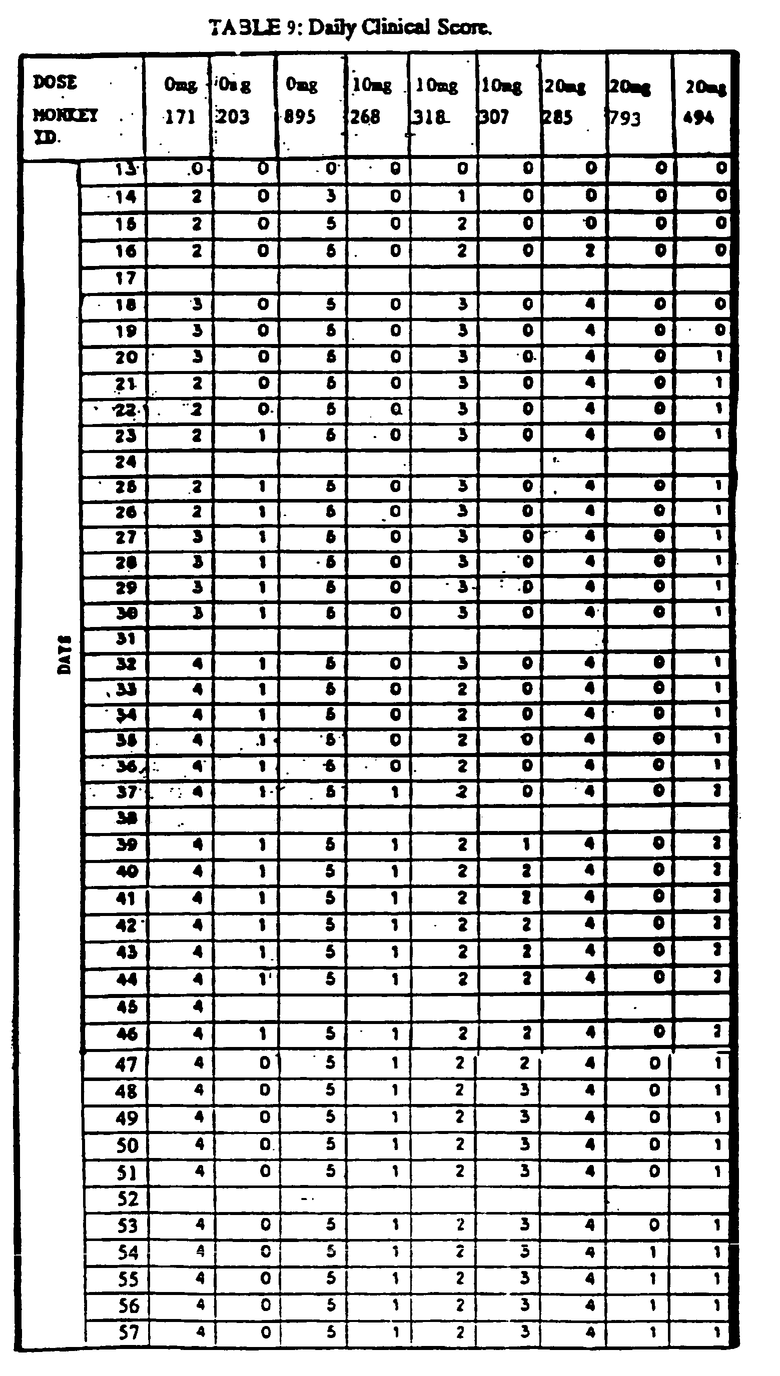

In the group that received the placebo, two females showed signs of illness on day 14 (scores 2 and 3). Female No. 895 was found dead on day 15 (score 5) and Female No. 171 was sacrificed on day 32 when score 4 was reached. Female No. 203 showed only mild signs of illness (score 1) on days 23-46.

In the 10 mg Copolymer 1 treatment group, Female No. 268 showed mild signs of illness on day 37, which continued until the end of the follow up period. Female No. 307 had a similar onset of symptoms, with mild signs on day 39 (score 1) but reached score 2 on days 40-46, and score 3 from day 46 until the end of the follow up period. Female No. 318 showed signs of illness (score 1) on day 14, reached score 2 on days 15-16, and score 3 on days 18-32, with an improvement starting on day 33 (score 2) until the end of the follow up period. Overall, she exhibited very mild symptoms during the entire follow up period.

In the group that was treated with 20 mg Copolymer 1, Female No. 285 showed a very acute onset of signs. On day 16, she reached score 2, and was euthanized two days later on day 18 (score 4). Female No. 793 had mild neurological symptoms (score 1) from day 54 until the end of follow up period- Female No. 494 showed mild signs from day 20 to 36, and reached score 2 from day 37 to 46, with an improvement (score 1) until the end of the follow up period.

A summary of the results is presented in Table 10 below.

**TABLE 10: Evaluation of Clinical Manifestations**

| Treatment Group | Incidence | Mean Score | Mean Maximal Score | Mean Disease Onset Day |
|---|---|---|---|---|
| Control | 3/3 | 2 . 9 ± 0 . 6 | 3.33 | 17 |
| 10 mg | 3/3 | 1 . 3 ± 0 . 56 | 2.33 | 30 |
| 20 mg | 3/3 | 1.6 ± 0.53 | 2.33 | 30 |

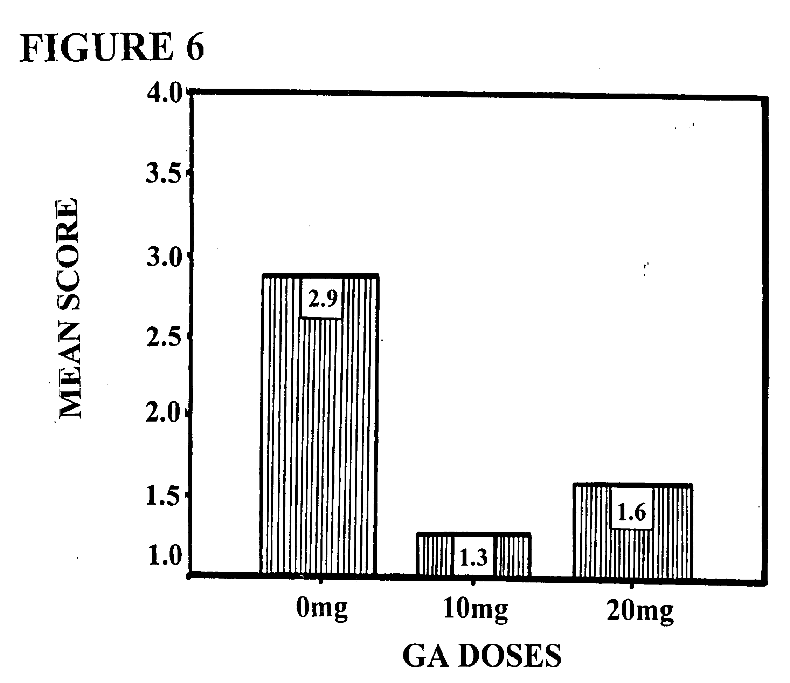

A graphic summary appears in Figure 6. Six females survived to the end of the follow up period and were sacrificed on day 57.

The phasic course of the disease was not clear as in Example 3B, with recurrence of symptoms and remission periods. Most of the animals developed acute or chronic disease. Acute onset of EAE appeared in the placebo group in two animals and in the 20 mg Copolymer 1 treatment group in one animal. In the group that was treated with 10 mg Copolymer 1, the pattern of disease was not as severe as in the other groups -- all three animals developed chronic disease and survived through the whole study. The difference between the placebo group and the 10 mg Copolymer 1 group, and between the placebo group and the 20 mg Copolymer 1 group was statistically significant (p < 0.0001). The difference is also significant between the 10 mg Copolymer 1 group and the 20 mg Copolymer 1 group (p < 0.03). From evaluation of the clinical manifestations summarized in Table 10, it is evident that Copolymer 1 treatment does not change the incidence'of the disease, but mainly affects the timing of the initial manifestation of the disease and the intensity of the symptoms. This data confirms the results of Example 3B, where the most effective dosage of Copolymer 1 was 10 mg.

### Example 3D: Treatment of Relapsing - Remitting (RR) MS Patients With Copolymer 1 Enteric-Coated Tablets.

1,650 RR MS patients are recruited and randomized 1:1:1 into treatment with 5 mg Copolymer 1, 50 mg Copolymer 1 or placebo, in a framework of a phase III clinical trial. Patients are followed every 2 months for a total duration of 56 weeks.

The primary end point of the study is the reduction of relapse rate by the treatment with Copolymer 1 enteric-coated tablets.

In the secondary outcome measures, the effect of Copolymer 1 on disease activity and burden of disease are monitored using magnetic resonance imaging (MRI).

In addition, the effect on brain atrophy, magnetization transfer (MT), spectroscopy (MRS) and disability are also being monitored, in order to demonstrate the effect of oral administration of Copolymer 1 in enteric-coated tablets on disease progression.

### DISCUSSION

When preparing oral formulations, characteristics such as desired site of action (e.g. gut), dosage amount, physical characteristics, and chemical characteristics, must be taken into consideration.

Copolymer 1 is a non-crystalline, highly porous, lyophilized material. It is only slightly soluble, and has poor mixing and very poor flow properties. In addition, Copolymer 1 is a proteinaceous material which is easily degraded by proteolytic enzymes in the gastrointestinal track. This disclosure provides Copolymer 1 formulations for oral administration which have pharmaceutical properties suitable for oral administration.

While microcrystalline cellulose has been used as a component in oral formulations, the formulations disclosed herein contain in excess of 50% by weight of microcrystalline cellulose. Such a composition, together with the disclosed milling/dry granulation manufacturing process results in oral formulations with excellent flow and mixing characteristics, improved dissolution and improved stability than that which could have been expected based on the properties-of Copolymer 1.

Indeed, based on the properties of Copolymer 1, it was unexpected that formulation with microcrystalline cellulose, particularly in excess of 50%, would have significantly improved pharmaceutical properties suitable for oral administration.

The advantageous properties of the disclosed formulation include that it allows for matching *in vitro* dissolution profiles of the 5 mg Copolymer 1 and the 50 mg Copolymer 1 tablets, despite a 4-fold difference in tablet weight as shown in Figure 3. Specifically, and unexpectedly, even thought the 50 mg Copolymer 1 tablet is four times the weight of the 5 mg Copolymer 1 tablet, the tablets have similar dissolution profiles.

## Claims

1. A pharmaceutical composition in solid form comprising as an active ingredient a therapeutically effective amount of glatiramer acetate, an amount of microcrystalline cellulose in excess of 50% by the weight of the composition, and an enteric coating.

2. The pharmaceutical composition of claim 1, wherein the amount of microcrystalline cellulose is at least 70% by weight.

3. The pharmaceutical composition of claim 1, wherein the amount of microcrystalline cellulose is from 60% to 90% by weight.

4. The pharmaceutical composition of claim 1, wherein the amount of microcrystalline cellulose is from 70% to 80% by weight.

5. The pharmaceutical composition of claim 1, wherein the microcrystalline cellulose has a moisture content of up to 5.0%.

6. The pharmaceutical composition of claim 1, wherein the microcrystalline cellulose has a moisture content of up to 1.5%.

7. The pharmaceutical composition of claim 1, further comprising a disintegrant.

8. The pharmaceutical composition of claim 7, wherein the disintegrant is selected from the group consisting of kaolin, starch, powdered sugar, sodium starch glycolate, crosscarmelose sodium, carboxymethyl cellulose, microcrystalline cellulose and sodium alginate.

9. The pharmaceutical composition of claim 8, wherein the disintegrant is a pregelatinized starch.

10. The pharmaceutical composition of claim 9, wherein the starch has a moisture content of up to 14%.

11. The pharmaceutical composition of claim 9, wherein the starch has a moisture content of up to 12%.

12. The pharmaceutical composition of claim 9, wherein the starch has a moisture content.of up to 7%.

13. The pharmaceutical composition of claim 9, wherein the starch has a moisture content of up to 5%.

14. The pharmaceutical composition of claim 1, further comprising a lubricant.

15. The pharmaceutical composition of claim 14, wherein the lubricant is selected from the group consisting of talc, sodium stearyl fumarate, magnesium stearate, calcium stearate, hydrogenated castor oil, hydrogenated soybean oil, and polyethylene glycol.

16. The pharmaceutical composition of claim 15, wherein the lubricant is magnesium stearate.

17. The pharmaceutical composition of claim 1, wherein the enteric coating is methacrylic acid copolymer.

18. The pharmaceutical composition of claim 1 , wherein the enteric coating is selected from the group consisting of cellulose acetate phthalate (CAP), hydroxypropyl methyl cellulose phthalate (HPMCP), carboxymethyl ethyl cellulose (CMEC), or amino-alkylmethacrylate copolymer.

19. The pharmaceutical composition of claim 1, further comprising a film coating under the enteric coating.

20. The pharmaceutical composition of claim 19, wherein the film coating is selected from the group consisting of hydroxyl propyl methyl cellulose (HPMC) and poly vinyl alcohol (PVA).

21. The pharmaceutical composition of claim 1, wherein the solid form is selected from the group consisting of a tablet, a hard gelatin capsule, a pellet and a particulate formulation.

22. The pharmaceutical composition of claim 21, wherein the solid form is a tablet and the effective amount of glatiramer acetate is from about 0.1 mg to about 300 mg.

23. The pharmaceutical composition of claim 22, wherein the effective amount of glatiramer acetate is from about 5 mg to about 100 mg.

24. The pharmaceutical .composition of claim 22, wherein the effective amount of glatiramer acetate is about 5 mg.

25. The pharmaceutical composition of claim 22, wherein the effective amount of glatiramer acetate is about 50 mg.

26. The pharmaceutical composition of claim 22, wherein the effective amount of.glatiramer acetate is about 300mg.

27. The pharmaceutical composition of claim 4, wherein the effective amount of glatiramer acetate is from about. 5 mg to about 100 mg.

28. The pharmaceutical composition of claim 4, wherein the effective amount of glatiramer acetate is about 5 mg.

29. The pharmaceutical composition of claim 4, wherein the effective amount of glatiramer acetate is about 50 mg.

30. The pharmaceutical composition of claim 4, wherein the effective amount of glatiramer acetate is from about 0.01 mg/kg to about 2 mg/kg.

31. The pharmaceutical composition of claim 4, wherein the effective amount of glatiramer acetate is from about 0.05 mg/kg to about 1 mg/kg.

32. The pharmaceutical composition of claim 1, further comprising a protease inhibitor.

33. The pharmaceutical composition of any of claims 1 to 32, for treating an autoimmune disease in a mammal.

34. The pharmaceutical composition of claim 33, wherein said autoimmune disease is multiple sclerosis.

35. The pharmaceutical composition of claim 33, wherein said autoimmune disease is selected from the group consisting of an arthritic condition, autoimmune hemolytic anemia, autoimmune oophoritis, autoimmune uveoretinitis, Crohn's disease, chronic immune thrombocytopenic purpura, colitis contact sensitivity disease, Graves disease, Guillain-Barre's syndrome, Hashimoto's disease, idiopathic myxedema, myasthenia gravis, psoriasis, pemphigus vulgaris, rheumatoid arthritis, GVHD and HVGD.

36. A process for manufacturing the composition of claim 1, comprising:
a) milling the glatiramer acetate,
b) dry mixing the milled glatiramer acetate with at least 50% by weight of microcrystalline cellulose, and
c) applying an enteric coating.

37. The process of claim 36, further comprising applying a film coating under the enteric coating.

38. The process of claim 36, wherein the enteric coating is applied using a rotating pan system.

39. A use of glatiramer acetate and microcrystalline cellulose for the preparation of a pharmaceutical composition in solid form, comprising in excess of 50% by weight of microcrystalline cellulose and an enteric coating, for treating an autoimmune disease in a mammal.

40. The use of claim 39, wherein the pharmaceutical composition comprises 5 mg to 100 mg of glatiramer acetate, and 70% - 80% by weight of microcrystalline cellulose.

41. The use of claim 39, wherein the pharmaceutical composition comprises 0.01 mg/kg to 2 mg/kg of a mammal of glatiramer acetate, and 70% - 80% by weight of microcrystalline cellulose.

42. The use of any of claims 39, 40 or 41, wherein said autoimmune disease is multiple sclerosis.

43. The use of any of claims 39, 40 or 41, wherein said autoimmune disease is selected from the group consisting of an arthritic condition, autoimmune hemolytic anemia, autoimmune oophoritis, autoimmune uveoretinitis, Crohn's disease, chronic immune thrombocytopenic purpura, colitis, contact sensitivity disease, Graves disease, Guillain-Barre's syndrome, Hashimoto's disease, idiopathic myxedema, myasthenia gravis, psoriasis, pemphigus vulgaris, rheumatoid arthritis, GVHD and HVGD.

## Patentansprüche

1. Arzneimittel in fester Form, umfassend als Wirkstoff eine therapeutisch wirksame Menge an Glatirameracetat, eine Menge an mikrokristalliner Cellulose in einem Überschuß von 50 Gew.-%, bezogen auf das Gewicht der Zusammensetzung, und einen magensaftresistenten Überzug.

2. Arzneimittel nach Anspruch 1, wobei die Menge an mikrokristalliner Cellulose mindestens 70 Gew.-% beträgt.

3. Arzneimittel nach Anspruch 1, wobei die Menge an mikrokristalliner Cellulose 60 bis 90 Gew.-% beträgt.

4. Arzneimittel nach Anspruch 1, wobei die Menge an mikrokristalliner Cellulose 70 bis 80 Gew.-% beträgt.

5. Arzneimittel nach Anspruch 1, wobei die mikrokristalline Cellulose einen Feuchtigkeitsgehalt von bis zu 5,0% aufweist.

6. Arzneimittel nach Anspruch 1, wobei die mikrokristalline Cellulose einen Feuchtigkeitsgehalt von bis zu 1,5% aufweist.

7. Arzneimittel nach Anspruch 1, weiter umfassend ein Sprengmittel.

8. Arzneimittel nach Anspruch 7, wobei das Sprengmittel ausgewählt ist aus Kaolin, Stärke, Puderzucker, Natriumstärkeglykolat, Crosscarmelosenatrium, Carboxymethylcellulose, mikrokristalliner Cellulose und Natriumalginat.

9. Arzneimittel nach Anspruch 8, wobei das Sprengmittel eine vorgelatinierte Stärke ist.

10. Arzneimittel nach Anspruch 9, wobei die Stärke einen Feuchtigkeitsgehalt von bis zu 14% aufweist.

11. Arzneimittel nach Anspruch 9, wobei die Stärke einen Feuchtigkeitsgehalt von bis zu 12% aufweist.

12. Arzneimittel nach Anspruch 9, wobei die Stärke einen Feuchtigkeitsgehalt von bis zu 7% aufweist.

13. Arzneimittel nach Anspruch 9, wobei die Stärke einen Feuchtigkeitsgehalt von bis zu 5% aufweist.

14. Arzneimittel nach Anspruch 1, weiter umfassend ein Gleitmittel.

15. Arzneimittel nach Anspruch 14, wobei das Gleitmittel ausgewählt ist aus Talkum, Natriumstearylfumarat, Magnesiumstearat, Calciumstearat, hydriertem Rizinusöl, hydriertem Sojaöl und Polyethylenglykol.

16. Arzneimittel nach Anspruch 15, wobei das Gleitmittel Magnesiumstearat ist.

17. Arzneimittel nach Anspruch 1, wobei der magensaftresistente Überzug Methacrylsäure-Copolymer ist.

18. Arzneimittel nach Anspruch 1, wobei der magensaftresistente Überzug ausgewählt ist aus Celluloseacetatphthalat (CAP), Hydroxypropylmethylcellulosephthalat (HPMCP), Carboxymethylethylcellulose (CMEC) oder Aminoalkylmethacrylat-Copolymer.

19. Arzneimittel nach Anspruch 1, weiter umfassend einen Filmüberzug unter dem magensaftresistenten Überzug.

20. Arzneimittel nach Anspruch 19, wobei der Filmüberzug ausgewählt ist aus Hydroxypropylmethylcellulose (HPMC) und Polyvinylalkohol (PVA).

21. Arzneimittel nach Anspruch 1, wobei die feste Form ausgewählt ist aus einer Tablette, einer Hartgelatinekapsel, einem Pellet und einer teilchenförmigen Formulierung.

22. Arzneimittel nach Anspruch 21, wobei die feste Form eine Tablette ist und die wirksame Menge an Glatirameracetat etwa 0,1 mg bis etwa 300 mg beträgt.

23. Arzneimittel nach Anspruch 22, wobei die wirksame Menge an Glatirameracetat etwa 5 mg bis etwa 100 mg beträgt.

24. Arzneimittel nach Anspruch 22, wobei die wirksame Menge an Glatirameracetat etwa 5 mg beträgt.

25. Arzneimittel nach Anspruch 22, wobei die wirksame Menge an Glatirameracetat etwa 50 mg beträgt.

26. Arzneimittel nach Anspruch 22, wobei die wirksame Menge an Glatirameracetat etwa 300 mg beträgt.

27. Arzneimittel nach Anspruch 4, wobei die wirksame Menge an Glatirameracetat etwa 5 mg bis etwa 100 mg beträgt.

28. Arzneimittel nach Anspruch 4, wobei die wirksame Menge an Glatirameracetat etwa 5 mg beträgt.

29. Arzneimittel nach Anspruch 4, wobei die wirksame Menge an Glatirameracetat etwa 50 mg beträgt.

30. Arzneimittel nach Anspruch 4, wobei die wirksame Menge an Glatirameracetat etwa 0,01 mg/kg bis etwa 2 mg/kg beträgt.

31. Arzneimittel nach Anspruch 4, wobei die wirksame Menge an Glatirameracetat etwa 0,05 mg/kg bis etwa 1 mg/kg beträgt.

32. Arzneimittel nach Anspruch 1, weiter umfassend einen Proteaseinhibitor.

33. Arzneimittel nach einem der Ansprüche 1 bis 32 zur Behandlung einer Autoimmunerkrankung bei einem Säuger.

34. Arzneimittel nach Anspruch 33, wobei die Autoimmunerkrankung Multiple Sklerose ist.

35. Arzneimittel nach Anspruch 33, wobei die Autoimmunerkrankung ausgewählt ist aus arthritischen Beschwerden, autoimmunhämolytischer Anämie, Autoimmun-Oophoritis, Autoimmun-Uveoretinitis, Morbus Crohn, chronischer immunthrombocytopenischer Purpura, Kolitis, Kontaktempfindlichkeit, Morbus Basedow, Guillain-Barre-Syndrom, Hashimoto-Thyreoiditis, idiopathischen Myxödemen, Erb-Goldflam-Syndrom, Psoriasis, Pemphigus vulgaris, rheumatoider Arthritis, Transplantat-Wirt-Reaktion (GvHR) und Wirt-Transplantat-Reaktion (HvGR).

36. Verfahren zur Herstellung der Zusammensetzung nach Anspruch 1, umfassend:
a) Mahlen des Glatirameracetats,
b) Trockenmischen des gemahlenen Glatirameracetats mit mindestens 50 Gew.-% mikrokristalliner Cellulose und
c) Aufbringen eines magensaftresistenten Überzugs.

37. Verfahren nach Anspruch 36, weiter umfassend das Aufbringen eines Filmüberzugs unter dem magensaftresistenten Überzug.

38. Verfahren nach Anspruch 36, wobei der magensaftresistente Überzug unter Verwendung eines Systems mit rotierender Pfanne aufgebracht wird.

39. Verwendung von Glatirameracetat und mikrokristalliner Cellulose zur Herstellung eines Arzneimittels in fester Form, umfassend mikrokristalline Cellulose in einem Überschuß von 50 Gew.-% und einen magensaftresistenten Überzug, zur Behandlung einer Autoimmunerkrankung bei einem Säuger.

40. Verwendung nach Anspruch 39, wobei das Arzneimittel 5 mg bis 100 mg Glatirameracetat und 70 bis 80 Gew.-% mikrokristalline Cellulose umfasst.

41. Verwendung nach Anspruch 39, wobei das Arzneimittel 0,01 mg/kg bis 2 mg/kg eines Säugers an Glatirameracetat und 70 bis 80 Gew.-% mikrokristalline Cellulose umfasst.

42. Verwendung nach einem der Ansprüche 39, 40 oder 41, wobei die Autoimmunerkrankung Multiple Sklerose ist.

43. Verwendung nach einem der Ansprüche 39, 40 oder 41, wobei die Autoimmunerkrankung ausgewählt ist aus arthritischen Beschwerden, autoimmunhämolytischer Anämie, Autoimmun-Oophoritis, Autoimmun-Uveoretinitis, Morbus Crohn, chronischer immunthrombocytopenischer Purpura, Kolitis, Kontaktempfindlichkeit, Morbus Basedow, Guillain-Barre-Syndrom, Hashimoto-Thyreoiditis, idiopathischen Myxödemen, Erb-Goldflam-Syndrom, Psoriasis, Pemphigus vulgaris, rheumatoider Arthritis, Transplantat-Wirt-Reaktion (GvHR) und Wirt-Transplantat-Reaktion (HvGR).

## Revendications

1. Composition pharmaceutique sous forme solide comprenant comme principe actif une quantité thérapeutiquement efficace d'acétate de glatiramer, une quantité de cellulose microcristalline dépassant 50 % en poids de la composition, et un enrobage entérique.

2. Composition pharmaceutique selon la revendication 1,
dans laquelle la quantité de cellulose microcristalline est d'au moins 70 % en poids.

3. Composition pharmaceutique selon la revendication 1,
dans laquelle la quantité de cellulose microcristalline est de 60 % à 90 % en poids.

4. Composition pharmaceutique selon la revendication 1,
dans laquelle la quantité de cellulose microcristalline est de 70 % à 80 % en poids.

5. Composition pharmaceutique selon la revendication 1,
dans laquelle la cellulose microcristalline possède un taux d'humidité allant jusqu'à 5,0 %.

6. Composition pharmaceutique selon la revendication 1,
dans laquelle la cellulose microcristalline possède un taux d'humidité allant jusqu'à 1,5 %.

7. Composition pharmaceutique selon la revendication 1, comprenant en outre un délitant.

8. Composition pharmaceutique selon la revendication 7,
dans laquelle le délitant est choisi dans le groupe constitué par le kaolin, l'amidon, le sucre en poudre, le glycolate d'amidon sodique, la croscarmellose sodique, la carboxyméthylcellulose, la cellulose microcristalline et l'alginate de sodium.

9. Composition pharmaceutique selon la revendication 8,
dans laquelle le délitant est de l'amidon prégélatinisé.

10. Composition pharmaceutique selon la revendication 9,
dans laquelle l'amidon possède un taux d'humidité allant jusqu'à 14 %.

11. Composition pharmaceutique selon la revendication 9,
dans laquelle l'amidon possède un taux d'humidité allant jusqu'à 12 %.

12. Composition pharmaceutique selon la revendication 9,
dans laquelle l'amidon possède un taux d'humidité allant jusqu'à 7 %.

13. Composition pharmaceutique selon la revendication 9,
dans laquelle l'amidon possède un taux d'humidité allant jusqu'à 5 %.

14. Composition pharmaceutique selon la revendication 1, comprenant en outre un lubrifiant.

15. Composition pharmaceutique selon la revendication 14,
dans laquelle le lubrifiant est choisi dans le groupe constitué par le talc, le fumarate de stéaryle sodique, le stéarate de magnésium, le stéarate de calcium, l'huile de ricin hydrogénée, l'huile de soja hydrogénée et le polyéthylène glycol.

16. Composition pharmaceutique selon la revendication 15,
dans laquelle le lubrifiant est le stéarate de magnésium.

17. Composition pharmaceutique selon la revendication 1,
dans laquelle l'enrobage entérique est un copolymère d'acide méthacrylique.

18. Composition pharmaceutique selon la revendication 1,
dans laquelle l'enrobage entérique est choisi dans le groupe constitué par l'acétophtalate de cellulose (CAP), le phtalate d'hydroxypropylméthylcellulose (HPMCP), la carboxyméthyléthylcellulose (CMEC), ou un copolymère de méthacrylate d'aminoalkyle.

19. Composition pharmaceutique selon la revendication 1, comprenant en outre une pellicule sous l'enrobage entérique.

20. Composition pharmaceutique selon la revendication 19,
dans laquelle la pellicule est choisie dans le groupe constitué par l'hydroxypropylméthylcellulose (HPMC) et l'alcool polyvinylique (PVA).

21. Composition pharmaceutique selon la revendication 1,
dans laquelle la forme solide est choisie dans le groupe constitué par un comprimé, une capsule de gélatine dure, une pastille et une formulation particulaire.

22. Composition pharmaceutique selon la revendication 21,
dans laquelle la forme solide est un comprimé et la quantité efficace d'acétate de glatiramer est d'environ 0,1 mg à environ 300 mg.

23. Composition pharmaceutique selon la revendication 22,
dans laquelle la quantité efficace d'acétate de glatiramer est d'environ 5 mg à environ 100 mg.

24. Composition pharmaceutique selon la revendication 22,
dans laquelle la quantité efficace d'acétate de glatiramer est d'environ 5 mg.

25. Composition pharmaceutique selon la revendication 22,
dans laquelle la quantité efficace d'acétate de glatiramer est d'environ 50 mg.

26. Composition pharmaceutique selon la revendication 22,
dans laquelle la quantité efficace d'acétate de glatiramer est d'environ 300 mg.

27. Composition pharmaceutique selon la revendication 4,
dans laquelle la quantité efficace d'acétate de glatiramer est d'environ 5 mg à environ 100 mg.

28. Composition pharmaceutique selon la revendication 4,
dans laquelle la quantité efficace d'acétate de glatiramer est d'environ 5 mg.

29. Composition pharmaceutique selon la revendication 4,
dans laquelle la quantité efficace d'acétate de glatiramer est d'environ 50 mg.

30. Composition pharmaceutique selon la revendication 4,
dans laquelle la quantité efficace d'acétate de glatiramer est d'environ 0,01 mg/kg à environ 2 mg/kg.

31. Composition pharmaceutique selon la revendication 4,
dans laquelle la quantité efficace d'acétate de glatiramer est d'environ 0,05 mg/kg à environ 1 mg/kg.

32. Composition pharmaceutique selon la revendication 1, comprenant en outre un inhibiteur de protéase.

33. Composition pharmaceutique selon l'une quelconque des revendications 1 à 32, destinée à traiter une maladie auto-immune chez un mammifère.

34. Composition pharmaceutique selon la revendication 33,
dans laquelle ladite maladie auto-immune est la sclérose en plaques.

35. Composition pharmaceutique selon la revendication 33,
dans laquelle ladite maladie auto-immune est choisie dans le groupe constitué par un état arthritique, l'anémie hémolytique auto-immune, l'ovarite auto-immune, l'uvéorétinite auto-immune, la maladie de Crohn, le purpura thrombocytopénique immun chronique, la colite, la maladie de sensibilité de contact, la maladie de Basedow, le syndrome de Guillain-Barre, la maladie d'Hashimoto, le myxoedème idiopathique, la myasthénie grave, le psoriasis, le pemphigus vulgaire, la polyarthrite rhumatoïde, la réaction de la greffe contre l'hôte(GVHD) et la réaction de l'hôte contre la greffe (HVGD).

36. Procédé de fabrication de la composition selon la revendication 1, comprenant :
a) le broyage de l'acétate de glatiramer,
b) le mélange à sec de l'acétate de glatiramer broyé avec au moins 50 % en poids de cellulose microcristalline, et
c) l'application d'un enrobage entérique.

37. Procédé selon la revendication 36, comprenant en outre l'application d'une pellicule sous l'enrobage entérique.

38. Procédé selon la revendication 36, dans lequel l'enrobage entérique est appliqué en utilisant un système de plateau tournant.

39. Utilisation d'acétate de glatiramer et de cellulose microcristalline pour la préparation d'une composition pharmaceutique sous forme solide, comprenant plus de 50 % en poids de cellulose microcristalline et un enrobage entérique, destinée à traiter une maladie auto-immune chez un mammifère.

40. Utilisation selon la revendication 39, dans laquelle la composition pharmaceutique comprend 5 mg à 100 mg d'acétate de glatiramer, et 70 % à 80 % en poids de cellulose microcristalline.

41. Utilisation selon la revendication 39, dans laquelle la composition pharmaceutique comprend 0,01 mg/kg à 2 mg/kg d'un mammifère d'acétate de glatiramer, et 70 % à 80 % en poids de cellulose microcristalline.

42. Utilisation selon l'une quelconque des revendications 39, 40 et 41, dans laquelle ladite maladie auto-immune est la sclérose en plaques.

43. Utilisation selon l'une quelconque des revendications 39, 40 et 41, dans laquelle ladite maladie auto-immune est choisie dans le groupe constitué par un état arthritique, l'anémie hémolytique auto-immune, l'ovarite auto-immune, l'uvéorétinite auto-immune, la maladie de Crohn, le purpura thrombocytopénique immun chronique, la colite, la maladie de sensibilité de contact, la maladie de Basedow, le syndrome de Guillain-Barre, la maladie d'Hashimoto, le myxoedème idiopathique, la myasthénie grave, le psoriasis, le pemphigus vulgaire, la polyarthrite rhumatoïde, la GVHD et la HVGD.
